# EUROPEAN PATENT SPECIFICATION

(11) **EP 4 255 376 B1**
(45) Date of publication and mention of the grant of the patent: **04.09.2024**
(21) Application number: 21806249.5
(22) Date of filing: 09.11.2021
(51) Int. Cl.: A61K 8/34, A61K 8/39, A61K 8/46, A61Q 19/10, A61K 8/04, C11D 3/20, C11D 3/00, C11D 3/37, C11D 17/00

(54) **SKIN CLEANSING COMPOSITION**
HAUTREINIGUNGSZUSAMMENSETZUNG
COMPOSITION DE NETTOYAGE DE LA PEAU

(30) Priority: 02.12.2020 WO PCT/CN2020/133451; 14.01.2021 EP 21151535
(43) Date of publication of application: 11.10.2023
(73) Proprietor: Unilever IP Holdings B.V., 3013 AL Rotterdam (NL); Unilever Global IP Limited, Wirral, Merseyside CH62 4ZD (GB)
(72) Inventor: CAO, Yixuan, Shanghai 200335 (CN); SHENG, Saihong, Shanghai 200335 (CN); SUN, Yingqing, Shanghai 200040 (CN); ZHU, Sunxin, Shanghai 200335 (CN)
(74) Representative: Fijnvandraat, Arnoldus
(86) International application number: PCT/EP2021/081009
(87) International publication number: WO 2022/117289

(56) References cited:
- WO-A2-01/79418
- CN-A- 109 464 300
- US-A1- 2005 049 169
- DATABASE GNPD [online] MINTEL; 17 January 2017 (2017-01-17), ANONYMOUS: "Foaming Body Wash", XP055818182, retrieved from https://www.gnpd.com/sinatra/recordpage/4556487/ Database accession no. 4556487

## Description

### Field of the invention

The present invention relates to a skin cleansing composition suitable for use in the automatic dispensing device. In particular such composition is capable of capable of producing fine, even and stable foam through automatic dispensing device after storage at high low temperature and leaving less residue at high temperature.

### Background of the invention

Due to the convenience and the contactless mode of automatic dispensing devices, they have been widely used to provide skin cleansing composition, on many occasions for example airport, hotel, home and et cetera. The temperature of the environment may vary a lot because of the regions and/or occasions. Such temperature may affect the performance of the skin cleansing composition in the automatic dispensing device. The present inventors have identified that there are some issues, for example the generated foam is not fine and dense after storage at low temperature, and more residues were generated around the nozzle of the devices at high temperature, increasing the risk of blocking nozzle.

CN109 464 300A appears to disclose post-foaming cleansing compositions comprising 10-20% of surfactant and 5-10% of propylene glycol and having good foam stability.

The present inventors have recognized that there is a need to develop a skin cleansing composition have a wide adaptability at different temperature. Thus, it can deliver good cleansing experience to consumer at low temperature while generated less residue at high temperature. It was surprisingly found that by including polypropylene glycol, and diol into skin cleansing composition comprising anionic surfactant with a specific weight ratio of the diol to the anionic surfactant, the composition is capable of producing fine, even and stable foam through automatic dispensing device, after storage at high low temperature and leaving less residue at high temperature.

### Summary of the invention

In a first aspect, the present invention is directed to a skin cleansing composition comprising anionic surfactant selected from alkyl sulfates, alkyl ether sulfates, or a mixture thereof; polypropylene glycol and diol, wherein the weight ratio of the diol to the anionic surfactant is at least 1:5.

All other aspects of the present invention will more readily become apparent upon considering the detailed description and examples which follow.

### Detailed description of the invention

Except in the examples, or where otherwise explicitly indicated, all numbers in this description indicating amounts of material or conditions of reaction, physical properties of materials and/or use may optionally be understood as modified by the word "about".

All amounts are by weight of the composition, unless otherwise specified.

It should be noted that in specifying any range of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) mutatis mutandis.

The anionic surfactant suitable for the present invention is selected from alkyl sulfates (i.e. sulphates), alkyl ether sulfates, or a mixture thereof. Preferably, the alkyl sulfates and alkyl ether sulfates has the respective formulae R₁OSO₃M and R₁O (C₂H₄O)ₓSO₃M, wherein each R₁ is alkyl or alkenyl of from 8 to 18 carbon atoms, x is an integer having a value of from 1 to 10, and each M is a cation such as ammonium, alkanolamines, such as triethanolamine, monovalent metals, such as sodium and potassium, and polyvalent metal cations, such as magnesium, and calcium. More preferably, each R₁ is alkyl or alkenyl of from 10 to 16 carbon atoms, x is an integer having a value of from 1 to 10, and each M is a cation of triethanolamine, sodium or potassium, Most preferably each R₁ and R₂ has 12 to 14 carbon atoms, in a linear rather than branched chain.

Typically, the anionic surfactants are selected from sodium lauryl sulphate and sodium lauryl ether sulphate, sodium trideceth sulfate, ammonium lauryl sulfate, ammonium lauryl ethyl sulfate or a mixture thereof. Preferred anionic surfactants are selected from sodium lauryl sulphate and sodium lauryl ether sulphate(n)EO, (where n is from 1 to 3); more preferably sodium lauryl ether sulphate(n)EO, (where n is from 1 to 3); most preferably sodium lauryl ether sulphate(n)EO where n=1.

Preferably, the total amount of the alkyl sulfates and the alkyl ether sulfates is preferably 1 to 25%, more preferably 2 to 20%, even more preferably 4 to 16%, and still even more preferably 6 to 14% by weight of the composition.

Preferably, the polypropylene glycol comprises 2 to 200, more preferably 3 to 50, even more preferably 4 to 20 polymerized units of propylene oxide. Preferably the polypropylene glycol has a number average molecular weight of 100 to 50,000, more preferably 200 to 5,000. Most preferably, the polypropylene glycol is polypropylene glycol-7(PPG-7).

Preferably, the polypropylene glycol is present in amount of 0.1 to 10%, more preferably 0.5 to 7%, more preferably 1 to 4% by weight of the composition.

Diol as used herein refers to alcohol having two hydroxyl group. It is preferable that the diol has from 2 to 10 carbon atoms, more preferably 3 to 6 carbon atoms, most preferably the diol has 3 to 4 carbon atoms.

Particularly preferred diol comprises ethylene glycol, propylene glycol, butylene glycol, diethylene glycol, dipropylene glycol, triethylene glycol, isoprene glycol, hexylene glycol, or a mixture thereof. More preferably, the diol comprises ethylene glycol, propylene glycol, butylene glycol, hexylene glycol, or a mixture thereof. Even more preferably, the diol comprises propylene glycol, butylene glycol, or a mixture thereof. Still even more preferably the diol comprises propylene glycol and most preferably the diol is propylene glycol. To sake of clarity, the diol is chemically different from the polypropylene glycol.

Typically, the amount of diol is 0.1 to 30%, preferably 0.5 to 20% and more preferably 1 to 15% and even more preferably from 2 and 10% by weight of the total weight of the composition.

To achieve a better foamability and/or sensory, the weight ratio of the diol to the total amount of the alkyl sulfates and the alkyl ether sulfates is preferably 1:5 to 50:1, more preferably 1:5 to 5:1, and even more preferably 1:4 to 2:1. To achieve a better foamability and/or sensory, the weight ratio of the polypropylene glycol to the total amount of the alkyl sulfates and the alkyl ether sulfates is preferably 1:50 to 10:1, more preferably 1:20 to 5:1, even more preferably 1:10 to 2:1, and still even more preferably 1:8 to 1:1.

The composition may additionally comprise a monomeric alcohol. Monomeric alcohol as used herein refers to alcohol which is not a polymer. The monomeric alcohol is chemically different from the diol and the polypropylene glycol. Preferably, the monomeric comprises glycerol (i.e., glycerine or glycerin), sorbitol, hydroxypropyl sorbitol, 1,2,6-hexanetriol, ethoxylated glycerol, propoxylated glycerol or a mixture thereof. More preferably, the comprises glycerin, sorbitol, or a mixture thereof. Still even more preferably the monomeric alcohol comprises glycerin and most preferably the monomeric alcohol is glycerin. Preferably, the monomeric alcohol has a molecular weight of 50 to 500, more preferably 60 to 300.

Preferably, the amount of monomeric alcohol is 0.1 to 30%, preferably 1 to 20% and more preferably 3 to 15% and even more preferably from 5 to 10% by weight of the total weight of the composition.

Preferably, the total amount of monomeric alcohol and the diol is 0.1 to 40%, preferably 1 to 20% and more preferably 3 to 15% and even more preferably from 6 and 12% by weight of the total weight of the composition. Preferably, the weight ratio of the total weight of the monomeric alcohol and the diol to the total weight of the alkyl sulfates and the alkyl ether sulfates is in the range of 1:10 to 10:1, more preferably 1:6 to 6:1 and even more preferably 1:2 to 2:1 to achieve a better foamability and/or sensory.

The composition may additionally comprise amphoteric surfactant. The amount of the amphoteric surfactant may range from 0.5 to 8%, preferably from 1 to 4% by weight of the total composition.

Examples of amphoteric surfactants comprises alkyl amine oxides, alkyl betaines, alkyl amidopropyl betaines, alkyl sulphobetaines (sultaines), alkyl glycinates, alkyl carboxyglycinates, alkyl amphoacetates, alkyl amphopropionates, alkylamphoglycinates, alkyl amidopropyl hydroxysultaines, and/or acyl taurates and acyl glutamates, wherein the alkyl and acyl groups have from 8 to 19 carbon atoms. Typical amphoteric surfactants for use in the composition of the present invention comprise lauryl amine oxide, cocodimethyl sulphopropyl betaine, lauryl betaine, cocamidopropyl betaine and/or sodium cocoamphoacetate. A particularly preferred amphoteric surfactant is cocamidopropyl betaine.

Water-insoluble skin benefit agents may also be formulated into the compositions as conditioners and moisturizers. Examples include silicone oils; hydrocarbons such as liquid paraffins, petrolatum, microcrystalline wax, and mineral oil; and vegetable triglycerides such as sunflower seed and cottonseed oils.

Some compositions may include thickeners. These may be selected from cellulosics, natural gums and acrylic polymers but not limited by this thickening agent types. Among the cellulosics are sodium carboxymethyl cellulose, hydroxyethyl cellulose, hydroxypropyl methylcellulose and combinations thereof. Suitable gums include xanthan, pectin, karaya, agar, alginate gums and combinations thereof. Among the acrylic thickeners are homopolymers and copolymers of acrylic and methacrylic acids including carbomers such as Carbopol 1382, Carbopol 982, Ultrez, Aqua SF-1 and Aqua SF-2 available from the Lubrizol Corporation. Amounts of thickener may range from 0.01 to 3% by weight of the active polymer (outside of solvent or water) in the compositions.

Preservatives can desirably be incorporated into the compositions of this invention to protect against the growth of potentially harmful microorganisms. Suitable traditional preservatives for compositions of this invention are alkyl esters of para-hydroxybenzoic acid. Other preservatives which have more recently come into use include hydantoin derivatives, propionate salts, and a variety of quaternary ammonium compounds. Particularly preferred preservatives are phenoxyethanol, methyl paraben, propyl paraben, imidazolidinyl urea, sodium dehydroacetate and benzyl alcohol. The preservatives should be selected having regard for the use of the composition and possible incompatabilities between the preservatives and other ingredients. Preservatives are preferably employed in amounts ranging from 0.01% to 2% by weight of the composition.

A variety of other optional materials may be formulated into the compositions. These may include: antimicrobials such as 2-hydroxy-4,2',4'-trichlorodiphenylether (triclosan), 2,6-dimethyl-4-hydroxychlorobenzene, and 3,4,4'-trichlorocarbanilide; scrub and exfoliating particles such as polyethylene and silica or alumina; cooling agents such as menthol; skin calming agents such as aloe vera; and colorants.

In addition, the compositions of the invention may further include 0.05 to 10% by weight of sequestering agents, such as tetra sodium ethylenediaminetetraacetate (EDTA), EHDP or mixtures; opacifiers and pearlizers such as ethylene glycol distearate, titanium dioxide or Lytron 621 (Styrene/Acrylate copolymer); all of which are useful in enhancing the appearance or properties of the product.

The composition may comprise water in amount of 10 to 95% by weight of the composition, more preferably from 20 to 88%, even more preferably from 35 to 82%, most preferably from 45 to 75% by weight of the composition.

Preferably, the composition has a viscosity of at least 5 mPa s, more preferably in the range 8 to 10000 mPa s, even more preferably 10 to 1000 mPa s, and most preferably 20 to 200 mPa s, when measured at 20 degrees C at a relatively high shear rate of about 20 s⁻¹. Preferably, the composition is in the form of fluid.

The skin cleansing composition (product) refers to a composition (product) suitable for topical application to human skin, preferably is a rinse-off product. The term "rinse-off" as used with reference to compositions herein means a composition that is applied to or rubbed on the skin, and then washed off. The term "skin" as used herein includes the skin on the face (except eye lids and lips), neck, chest, abdomen, back, arms, under arms, hands, and legs. Preferably "skin" means includes the skin on the face (except eye lids and lips) and under arms, more preferably skin means skin on the face other than lips and eyelids.

The following examples are provided to facilitate an understanding of the invention. The examples are not intended to limit the scope of the claims.

### Examples

### Example 1

This example demonstrates the effect of propylene glycol, diol on the foaming performance.

**Table 1**

| Ingredient | Samples (wt%*) | | | | | | | |
|---|---|---|---|---|---|---|---|---|
| | A | 1 | 2 | B | C | 3 | 4 | D |
| Water | To 100 | To 100 | To 100 | To 100 | To 100 | To 100 | To 100 | To 100 |
| Sodium Laureth Sulfate | 9.00 | 9.00 | 9.00 | 9.00 | 9.00 | 9.00 | 9.00 | 9.00 |
| Cocamidopropyl Betaine | 1.59 | 1.59 | 1.59 | 1.59 | 1.59 | 1.59 | 1.59 | 1.59 |
| Glycerin | 3.00 | 6.00 | 6.00 | 6.00 | 9.00 | - | 9.00 | 20.00 |
| Propylene Glycol | 1.50 | 3.00 | 3.00 | 3.00 | - | 9.00 | 3.00 | - |
| PPG-7 | 0.80 | 0.80 | 1.20 | - | 1.20 | 1.20 | 0.80 | 1.20 |
| PEG-8 | - | - | - | 1.20 | - | - | - | - |
| Tetrasodium EDTA | 0.05 | 0.05 | 0.05 | 0.05 | 0.05 | 0.05 | 0.05 | 0.05 |
| Citric acid | 0.30 | 0.30 | 0.30 | 0.30 | 0.30 | 0.30 | 0.30 | 0.30 |
| Preservative | 0.55 | 0.55 | 0.55 | 0.55 | 0.55 | 0.55 | 0.55 | 0.55 |
| Fragrance | 0.56 | 0.56 | 0.56 | 0.56 | 0.56 | 0.56 | 0.56 | 0.56 |

| | | | | | | | | |
|---|---|---|---|---|---|---|---|---|
| *The level of the ingredients refers the level of active. | | | | | | | | |

A series of skin cleansing compositions were formulated according to Table 1 by following standard procedure.

The performances of foaming for all samples at low temperature were evaluated by the procedure as follows. Each sample was filled into identical automatic-dosing device and stored at 5 °C for 15 days. Identical amounts of samples were then pumped out from the devices onto a watch glass. The foam was observed and the performance of foamability for each sample was evaluated from the perspective of richness and fineness. It is marked as "not acceptable" for foam having a lot of large bubbles, "good" for foam being fine and dense, and "acceptable" for foam in the middle.

The performances of the samples at high temperature were evaluated by the following procedure. Each sample was filled into identical automatic-dosing device. The samples were pumped out from the device into glass beakers to make the glass beakers full of foam. Then, the glass beakers with samples were placed into oven at 50°C for 7 hours. Then, these glass beakers were taken out and observed. It is marked as "not acceptable" for sample having a lot of residues on the wall of the glass beaker, "good" for sample leaving no noticeable residue on the wall of the glass beaker, and "acceptable" for sample in the middle.

The performances of all samples were recorded in Table 2.

**Table 2**

| Performance | Samples | | | | | | | |
|---|---|---|---|---|---|---|---|---|
| | A | 1 | 2 | B | C | 3 | 4 | D |
| Foamability at 5° C | × | √ | √√ | √ | √√ | √√ | √ | × |
| Performance at 45° C | × | √ | √√ | × | × | √√ | √√ | √√ |

| | | | | | | | | |
|---|---|---|---|---|---|---|---|---|
| x: not acceptable; √: acceptable; √√: good | | | | | | | | |

It was surprisingly found that by including polypropylene glycol, and diol into skin cleansing composition comprising anionic surfactant with a specific weight ratio of the diol to the anionic surfactant, the composition is capable of producing fine, even and stable foam through automatic dispensing device, even after storage at low temperature, as well as leaving much less residue at high temperature.

## Claims

1. A skin cleansing composition comprising:
(a) anionic surfactant selected from alkyl sulfates, alkyl ether sulfates, or a mixture thereof;
(b) polypropylene glycol; and
(c) diol;
wherein the weight ratio of the diol to the anionic surfactant is at least 1:5.

2. The composition according to claim 1 wherein the total amount of the alkyl sulfates and the alkyl ether sulfates is 1 to 25%, preferably 4 to 16% by weight of the composition.

3. The composition according to claim 1 or 2 wherein the anionic surfactant is selected from sodium lauryl sulphate and sodium lauryl ether sulphate, sodium trideceth sulfate, ammonium lauryl sulfate, ammonium lauryl ethyl sulfate or a mixture thereof.

4. The composition according any one of the preceding claims wherein polypropylene glycol is polypropylene glycol-7.

5. The composition according to any one of the preceding claims wherein the polypropylene glycol is present in amount of 0.1 to 10%, preferably 1 to 4% by weight of the composition.

6. The composition according to any one of the preceding claims wherein the diol comprises ethylene glycol, propylene glycol, butylene glycol, hexylene glycol, or a mixture thereof, preferably the diol comprises propylene glycol.

7. The composition according to any one of the preceding claims wherein the amount of diol is 0.1 to 30%, preferably 1 to 15% by weight of the total weight of the composition.

8. The composition according to any one of the preceding claims wherein the weight ratio of the diol to the total amount of the alkyl sulfates and the alkyl ether sulfates is preferably 1:5 to 50:1, more preferably 1:4 to 2:1.

9. The composition according to any one of the preceding claims wherein the weight ratio of the polypropylene glycol to the total amount of the alkyl sulfates and the alkyl ether sulfates is preferably 1:50 to 10:1, more preferably 1:10 to 2:1.

10. The composition according to any one of the preceding claims wherein the composition further comprises a monomeric alcohol, preferably the monomeric alcohol is glycerin.

11. The composition according to claim 10 wherein the weight ratio of the total weight of the monomeric alcohol and the diol to the total weight of the alkyl sulfates and the alkyl ether sulfates is in the range of 1:10 to 10:1, preferably 1:2 to 2:1.

12. The composition according to claim 10 or 11 wherein the total amount of monomeric alcohol and the diol is 0.1 to 40%, preferably 3 to 15% by weight of the total weight of the composition.

13. The composition according to any one of the preceding claims wherein the composition comprises amphoteric surfactant, preferably the amphoteric surfactant is cocamidopropyl betaine.

14. The composition according to claim 13 wherein the amount of the amphoteric surfactant ranges from 0.5 to 8% by weight of the total composition.

15. The composition according to any one of the preceding claims wherein the composition comprises 35 to 82% of water by weight of the composition.

## Patentansprüche

1. Hautreinigungszusammensetzung, umfassend:
(a) anionisches Tensid, ausgewählt unter Alkylsulfaten, Alkylethersulfaten oder einer Mischung davon;
(b) Polypropylenglykol; und
(c) Diol;
wobei das Gewichtsverhältnis Diol zu anionischem Tensid mindestens 1:5 beträgt.

2. Zusammensetzung nach Anspruch 1, wobei die Gesamtmenge der Alkylsulfate und der Alkylethersulfate 1 bis 25%, bevorzugt 4 bis 16%, bezogen auf das Gewicht der Zusammensetzung, beträgt.

3. Zusammensetzung nach Anspruch 1 oder 2, wobei das anionische Tensid unter Natriumlaurylsulfat und Natriumlaurylethersulfat, Natriumtridecethsulfat, Ammoniumlaurylsulfat, Ammoniumlaurylethylsulfat oder einer Mischung davon ausgewählt ist.

4. Zusammensetzung nach irgendeinem der vorhergehenden Ansprüche, wobei das Polypropylenglykol Polypropylenglykol-7 ist.

5. Zusammensetzung nach irgendeinem der vorhergehenden Ansprüche, wobei das Polypropylenglykol in einer Menge von 0,1 bis 10%, bevorzugt 1 bis 4%, bezogen auf das Gewicht der Zusammensetzung, vorliegt.

6. Zusammensetzung nach irgendeinem der vorhergehenden Ansprüche, wobei das Diol Ethylenglykol, Propylenglykol, Butylenglykol, Hexylenglykol oder eine Mischung davon umfasst, wobei das Diol bevorzugt Propylenglykol umfasst.

7. Zusammensetzung nach irgendeinem der vorhergehenden Ansprüche, wobei die Menge an Diol 0,1 bis 30%, bevorzugt 1 bis 15%, bezogen auf das Gesamtgewicht der Zusammensetzung, beträgt.

8. Zusammensetzung nach irgendeinem der vorhergehenden Ansprüche, wobei das Gewichtsverhältnis Diol zu der Gesamtmenge der Alkylsulfate und der Alkylethersulfate bevorzugt 1:5 bis 50:1, bevorzugter 1:4 bis 2:1, beträgt.

9. Zusammensetzung nach irgendeinem der vorhergehenden Ansprüche, wobei das Gewichtsverhältnis Polypropylenglykol zu der Gesamtmenge der Alkylsulfate und der Alkylethersulfate bevorzugt 1:50 bis 10:1, bevorzugter 1:10 bis 2:1, beträgt.

10. Zusammensetzung nach irgendeinem der vorhergehenden Ansprüche, wobei die Zusammensetzung außerdem einen monomeren Alkohol umfasst, wobei der monomere Alkohol bevorzugt Glycerin ist.

11. Zusammensetzung nach Anspruch 10, wobei das Gewichtsverhältnis Gesamtgewicht des monomeren Alkohols und des Diols zu Gesamtgewicht der Alkylsulfate und der Alkylethersulfate in dem Bereich von 1:10 bis 10:1, bevorzugt 1:2 bis 2:1, liegt.

12. Zusammensetzung nach Anspruch 10 oder 11, wobei die Gesamtmenge des monomeren Alkohols und des Diols 0,1 bis 40%, bevorzugt 3 bis 15 Gew.-%, bezogen auf das Gesamtgewicht der Zusammensetzung, beträgt.

13. Zusammensetzung nach irgendeinem der vorhergehenden Ansprüche, wobei die Zusammensetzung amphoteres Tensid umfasst, wobei das amphotere Tensid bevorzugt Cocamidopropylbetain ist.

14. Zusammensetzung nach Anspruch 13, wobei die Menge des amphoteren Tensids in dem Bereich von 0,5 bis 8 Gewichts-% der gesamten Zusammensetzung liegt.

15. Zusammensetzung nach irgendeinem der vorhergehenden Ansprüche, wobei die Zusammensetzung 35 bis 82% Wasser, bezogen auf das Gewicht der Zusammensetzung, umfasst.

## Revendications

1. Composition nettoyante pour la peau comprenant :
(a) un tensioactif anionique choisi parmi les alkylsulfates, les alkyléthersulfates ou un mélange de ceux-ci ;
(b) un polypropylène glycol ; et
(c) un diol ;
dans laquelle le rapport pondéral du diol au tensioactif anionique est d'au moins 1:5.

2. Composition selon la revendication 1 dans laquelle la quantité totale des alkylsulfates et des alkyléthersulfates est de 1 à 25 %, de préférence de 4 à 16 % en poids de la composition.

3. Composition selon la revendication 1 ou 2 dans laquelle le tensioactif anionique est choisi parmi le laurylsulfate de sodium et le lauryléthersulfate de sodium, le tridécethsulfate de sodium, le laurylsulfate d'ammonium, le lauryléthylsulfate d'ammonium ou un mélange de ceux-ci.

4. Composition selon l'une quelconque des revendications précédentes dans laquelle le polypropylène glycol est le polypropylène glycol-7.

5. Composition selon l'une quelconque des revendications précédentes dans laquelle le polypropylène glycol est présent en une quantité de 0,1 à 10 %, de préférence 1 à 4 % en poids de la composition.

6. Composition selon l'une quelconque des revendications précédentes dans laquelle le diol comprend l'éthylène glycol, le propylène glycol, le butylène glycol, l'hexylène glycol, ou un mélange de ceux-ci, de préférence le diol comprend le propylène glycol.

7. Composition selon l'une quelconque des revendications précédentes dans laquelle la quantité de diol est de 0,1 à 30 %, de préférence de 1 à 15 % en poids du poids total de la composition.

8. Composition selon l'une quelconque des revendications précédentes dans laquelle le rapport pondéral du diol à la quantité totale des alkylsulfates et des alkyléthersulfates est de préférence de 1:5 à 50:1, plus préférablement de 1:4 à 2:1.

9. Composition selon l'une quelconque des revendications précédentes dans laquelle le rapport pondéral du polypropylène glycol à la quantité totale des alkylsulfates et des alkyléthersulfates est de préférence de 1:50 à 10:1, plus préférablement de 1:10 à 2:1.

10. Composition selon l'une quelconque des revendications précédentes dans laquelle la composition comprend en outre un alcool monomère, de préférence l'alcool monomère est la glycérine.

11. Composition selon la revendication 10 dans laquelle le rapport pondéral du poids total de l'alcool monomère et du diol au poids total des alkylsulfates et des alkyléthersulfates est compris dans la plage de 1:10 à 10:1, de préférence 1:2 à 2:1.

12. Composition selon la revendication 10 ou 11 dans laquelle la quantité totale d'alcool monomère et du diol est de 0,1 à 40 %, de préférence de 3 à 15 % en poids du poids total de la composition.

13. Composition selon l'une quelconque des revendications précédentes dans laquelle la composition comprend un tensioactif amphotère, de préférence le tensioactif amphotère est la cocamidopropylbétaïne.

14. Composition selon la revendication 13 dans laquelle la quantité de tensioactif amphotère est comprise dans la plage allant de 0,5 à 8 % en poids de la composition totale.

15. Composition selon l'une quelconque des revendications précédentes dans laquelle la composition comprend de 35 à 82 % d'eau en poids de la composition.
